(19) Europäisches Patentamt

European Patent Office

Office européen des brevets

(11) **EP 1 495 671 A1**

(12) **DEMANDE DE BREVET EUROPEEN**

(43) Date de publication:
**12.01.2005 Bulletin 2005/02**

(51) Int Cl.$^7$: **A01H 1/08**

(21) Numéro de dépôt: **03291693.4**

(22) Date de dépôt: **08.07.2003**

(84) Etats contractants désignés:
**AT BE BG CH CY CZ DE DK EE ES FI FR GB GR
HU IE IT LI LU MC NL PT RO SE SI SK TR**
Etats d'extension désignés:
**AL LT LV MK**

(71) Demandeur: **INSTITUT NATIONAL DE LA
RECHERCHE AGRONOMIQUE
75007 Paris (FR)**

(72) Inventeurs:
• **Bordes, Jacques
63000 Clermont-Ferrand (FR)**

• **Pollacsek, Maurice
63000 Clermont-Ferrand (FR)**
• **Beckert, Michel
63800 Cournon d'Auvergne (FR)**

(74) Mandataire: **Corizzi, Valérie et al
Cabinet ORES,
36, rue de Saint Pétersbourg
75008 Paris (FR)**

(54) **Obtention de plantes double-haploides par gynogenese chez le mais**

(57) L'invention concerne l'utilisation de plantes de la lignée PK6 de maïs, ou d'une lignée dérivée, comme inducteurs d'haploïdes gynogénétiques chez le maïs.

## EP 1 495 671 A1

**Description**

La présente invention concerne l'obtention de plants double-haploïdes de maïs par gynogenèse *in situ.*

L'établissement de lignées pures par autofécondations ou croisements consanguins répétés, constitue une pratique fondamentale en sélection et création variétale. L'une des contraintes majeures rencontrées lors de l'établissement de lignées pures réside dans le temps nécessaire (généralement 8 à 10 générations) pour obtenir des individus présentant un haut niveau d'homozygotie.

La création de plantes double-haploïdes (DH) permet d'envisager une simplification considérable des pratiques de sélection et de création variétale.

Les plantes DH, qui sont obtenues par doublement chromosomique à partir de plantes haploïdes, ont la particularité d'être homozygotes pour l'ensemble de leur génome. Leur utilisation en sélection semencière permet de diminuer considérablement le temps d'obtention de nouvelles variétés et de fixer rapidement les meilleurs génotypes. Elle permet notamment de déceler rapidement la présence éventuelle d'allèles défavorables récessifs.

Les méthodes les plus fréquemment utilisées pour obtenir des plantes haploïdes ou double-haploïdes sont basées sur l'androgenèse ou sur la gynogenèse.

L'androgenèse consiste en la régénération de plantes entières à partir de cellules sexuelles mâles, les microspores. Dans certaines conditions de culture *in vitro*, ces microspores sont capables de se développer et de se différencier pour donner des embryons haploïdes. Ces embryons aboutissent à la production de plantes haploïdes ou, après doublement chromosomique spontané ou artificiel (par exemple par traitement à la colchicine), de plantes double-haploïdes.

La gynogenèse consiste en la régénération de plantes entières à partir d'ovules ou d'ovaires non fécondés. Comme l'androgenèse, elle peut s'effectuer par culture in vitro.

Toutefois, elle peut également intervenir spontanément *in vivo,* à la suite d'une anomalie du déroulement du processus de fécondation.

Il sera brièvement rappelé que chez les angiospermes, il se produit normalement une double fécondation. Le grain de pollen produit deux cellules gamétiques mâles. L'une féconde l'ovule pour former le zygote diploïde qui produira l'embryon ; l'autre fusionne avec le noyau du sac embryonnaire, déjà habituellement diploïde, pour former une cellule triploïde qui produira l'albumen.

Dans certains cas, la formation du zygote ne se produit pas, mais des divisions cellulaires de l'ovule sont toutefois initiées, aboutissant à un embryon haploïde capable de donner naissance à une plantule dont le génome haploïde est entièrement d'origine maternelle (SARKAR et COE, Genetics, vol 54, pp. 453-464, 1966).

Le développement de plantules haploïdes a été observé chez de nombreuses espèces (KIMBER et RILEY, Bot rev, 29, 480-531, 1963 ; MAGOON et KHANNA, Caryologia, 16, 191-235, 1963). Chez le maïs, l'existence de plants haploïdes a été rapportée dès 1929 (RANDOLPH, Proc. Natl. Acad. Sci., 18, 222-229, 1932).

Chez le maïs, ces haploïdes gynogénétiques sont viables et ont un développement végétatif normal. Les plants mâles sont généralement stériles ; cependant, certaines des fleurs femelles sont fertiles et peuvent être fécondées par du pollen de plantes diploïdes.

Le doublement chromosomique n'intervient spontanément que très rarement. Toutefois, il peut être obtenu artificiellement : le traitement des plantules par la colchicine permet d'obtenir des plantules double-haploïdes dans plus de 50% des cas (DEIMLING et al., Vortr. Pflanzenzüchtg. Vol 38, 203-224, 1997). Ce traitement permet également de restaurer la fertilité mâle dans 30 à 60 % des cas (BORDES et al., Agronomie, vol 17, pp. 291-297).

La gynogenèse *in vivo* (également dénommée gynogenèse *in situ*) présente l'avantage potentiel d'être plus simple à mettre en oeuvre que les techniques *in vitro,* et d'être moins dépendante que celles-ci du génotype du matériel de départ. Cependant, la très faible fréquence et le caractère aléatoire de l'apparition spontanée d'haploïdes font obstacle à l'utilisation de cette technique en sélection variétale.

Il a été observé que la fréquence d'induction d'haploïdes gynogénétiques chez le maïs était liée au fond génétique des deux plantes mises en présence (CHASE, Agron. J., vol 44, pp. 263-267, 1952), et dépendait plus particulièrement de la plante mâle donneuse de pollen.

Des plantes dont le pollen est capable d'induire la formation d'haploïdes gynogénétiques in situ à une fréquence significativement plus élevée que celle observée dans les populations naturelles de maïs, ont été identifiées. Ces plantes sont dites : « inductrices » d'haploïdes gynogénétiques.

La première lignée inductrice d'haploïdes gynogénétiques mise en évidence chez le maïs est la lignée « Stock 6 », pour laquelle un taux d'induction d'haploïdes gynogénétiques pouvant aller jusqu'à 3,2% en auto-pollinisation a été rapporté (COE, Am. Nat., 93, 381-382, 1959).

Dans le but d'améliorer le taux d'induction d'haploïdes, la lignée Stock 6 a servi de base à l'élaboration de nouvelles lignées inductrices, parmi lesquelles on citera notamment la lignée inductrice WS14, pour laquelle un taux d'induction d'haploïdes de 1,2 à 5,5 fois supérieur à celui de Stock 6 a été obtenu (LASHHERMES et BECKERT, Theor. Appl. Genet., 76, 405-410, 1988) et la lignée inductrice FIGH 1, descendante de la lignée WS14 (BORDES et al.,

Agronomie, 17, 291-297, 1997).

Un autre problème posé par la mise en oeuvre de la gynogenèse *in situ* résulte dans la différenciation des individus haploïdes résultant du développement gynogénétique et des individus hybrides résultant des processus de fécondation et de développement embryonnaire normaux.

Il a été proposé d'utiliser dans ce but des marqueurs de coloration du grain. A titre d'exemple, on citera le « Purple Embryo Marker » décrit par CHASE et NANDA (Maize Gen. Coop., News Letter, 39, 59-60, 1965). Ce marqueur dominant se traduit par une coloration violet pourpre des grains. Lorsqu'il est présent dans le génome du mâle inducteur, les grains résultant d'une fécondation normale présentent un embryon et un endosperme colorés ; en revanche, chez les grains résultant du développement gynogénétique, seul l'endosperme est coloré. Un autre système de marquage du grain, décrit par COE et SARKAR, (J. of Heredity, vol 55, pp.231-233, 1964), associe un parent femelle possédant un marqueur de coloration (CC) et un mâle inducteur possédant un allèle inhibiteur de cette coloration (CICI). Chez les grains issus d'une fécondation normale, ni l'embryon ni l'endosperme ne sont colorés ; chez les grains résultant du développement gynogénétique, seul l'embryon est coloré.

Il subsiste différents problèmes liés à l'utilisation de ces marqueurs pour l'identification des plants haploïdes. Notamment, l'expression de ces marqueurs est fortement influencée par le génotype du parent femelle, ou même par le processus général de maturation du grain. Cette perturbation de l'expression est susceptible d'entraîner une évaluation incorrecte de la ploïdie des individus isolés.

On effectue donc fréquemment une sélection au stade de la plantule en complément de la sélection au stade du grain, ou à la place de celle-ci. Cette sélection peut se faire sur la base de l'observation d'une morphologie caractéristique des plants haploïdes (feuilles plus étroites, plus claires, striées, ports érigés.....). Il a également été proposé d'utiliser des marqueurs récessifs présents dans le génome du parent femelle. On citera par exemple le marqueur récessif « liguleless », qui se traduit par une absence de ligules au niveau des feuilles (EMERSON., *Nebraska. Agric. Exp. Stn. Annu. Rep.,* 1912a, vol 25, pp. 81-88; EMERSON., *Am. Breeders'. Assoc. Annu. Rep.,* 1912b, vol 8, pp. 385-399) et le marqueur récessif « glossy », qui se traduit par un aspect brillant de la surface des feuilles des jeunes plants, au lieu de la glaucescence bleutée observée chez les plants normaux (BORDES et al., Agronomie, 1997, vol 17, pp. 291-297). Lorsque ces marqueurs sont présents dans le génome du parent femelle, ils permettent de différencier les plantules haploïdes présentant le marqueur récessif, par exemple le caractère « glossy », des plantules hybrides qui ne le présentent pas. Toutefois, l'utilisation de ce type de marqueurs récessifs peut nécessiter, préalablement à l'induction de la gynogenèse, l'introduction du marqueur récessif dans le génome de la lignée destinée à être utilisée comme parent femelle, si celle-ci ne le possède pas déjà.

Les Inventeurs ont développé une nouvelle lignée inductrice, dénommée ci-après PK6, qui permet l'induction d'haploïdes gynogénétiques à partir de plantes femelles de génotypes très variés, avec un taux d'induction très supérieur à celui obtenu avec les lignées inductrices de l'art antérieur.

La lignée PK6 a été déposée le 19/02/2003 selon le traité de Budapest auprès de la National Collection of Industrial, Food and Marine Bacteria (NCIMB, 23 St Machar Drive, Aberdeen, Scottland, AB24 3 RY, Scotland, Royaume-Uni) sous le numéro d'accès NCIMB 41160.

La présente invention a pour objet l'utilisation d'une plante de la lignée PK6, ou d'une lignée dérivée, en tant qu'inducteur d'haploïdes gynogénétiques chez le maïs.

La présente invention a en particulier pour objet un procédé d'obtention d'haploïdes de maïs par pollinisation d'un parent femelle avec le pollen d'un parent inducteur d'haploïdes gynogénétiques et sélection des haploïdes issus de cette pollinisation, lequel procédé est caractérisé en ce que le parent inducteur d'haploïdes gynogénétiques appartient à la lignée PK6 ou à une lignée dérivée.

On entend par « lignée dérivée » de PK6, toute lignée issue de PK6 et ayant hérité les propriétés d'induction d'haploïdes gynogénétiques de celle-ci. Ceci inclut en particulier des lignées résultant de l'introduction d'un caractère d'intérêt, (notamment un marqueur permettant la différenciation des plants haploïdes et des plants diploïdes) dans le génome de PK6. L'introduction de ce caractère d'intérêt peut s'effectuer par exemple par les techniques classiques d'introgression ou de transgenèse.

A titre d'exemple de lignée dérivée de PK6, on citera la lignée PK6-cherry. Cette lignée résulte de l'introduction dans la lignée PK6 de l'allèle dominant « R-cherry », dont la présence se manifeste au stade plantule par une forte coloration rouge du joint foliaire (à la limite de la ligule) de la première et parfois de la seconde feuille. L'absence de cette coloration rouge permet de distinguer les plantules haploïdes.

La lignée PK6 cherry a été déposée le 19/02/2003 selon le traité de Budapest auprès de la National Collection of Industrial, Food and Marine Bacteria (NCIMB, 23 St Machar Drive, Aberdeen, Scottland, AB24 3 RY, Scotland, Royaume-Uni) sous le numéro d'accès NCIMB 41161.

Dans le cadre de la mise en oeuvre de la présente invention, la sélection des haploïdes peut s'effectuer par des méthodes connues en elles-mêmes, par exemple sur la base de critères morphologiques caractéristiques de l'haploïdie, ou sur l'observation d'un caractère récessif du parent femelle (par exemple liguleless, glossy, etc.).

Lorsque la plante inductrice appartient à la lignée PK6-cherry, cette étape de sélection des plantules haploïdes pourra avantageusement être mise en oeuvre en détectant l'absence de coloration rouge du joint foliaire. Le tri peut se faire visuellement lorsque la plantule a développé 3 à 4 feuilles visibles, soit à partir de 10 à 20 jours après le semis, selon la saison.

La présente invention a également pour objet un procédé d'obtention de plantes double haploïdes caractérisé en ce qu'il comprend la culture des haploïdes obtenus à l'issue du procédé d'haploïdisation conforme à l'invention, et la mise en oeuvre d'une étape de doublement chromosomique de ces haploïdes.

La technique utilisable pour effectuer le doublement chromosomique d'haploïdes issus de la gynogenèse in situ utilise un traitement à la colchicine. Ce traitement est effectué par mise en contact de la plantule haploïde (généralement par trempage) avec une solution de colchicine à 1,5 g/l. Avantageusement, ce traitement est effectué lorsque la plantule a atteint un stade de développement compris entre 5 et 7 feuilles visibles.

A l'issue du traitement de doublement chromosomique, les plantes traitées sont remises en culture jusqu'à maturation des organes floraux et l'on opère alors une autofécondation de ces plantes.

La présente invention sera mieux comprise à l'aide du complément de description qui va suivre, qui se réfère à des exemples non-limitatifs illustrant les caractéristiques des lignées PK6 et PK6-cherry et leur utilisation selon l'invention, pour l'obtention de plantes double-haploïdes.

## EXEMPLE 1: CARACTERISTIQUES DES LIGNEES PK6 ET PK6-CHERRY

### Description détaillée de PK6

| Description de PK6 | | | | |
|---|---|---|---|---|
| Albumen | | Allèle        y présence de carotène<br>Allèle fl0⁺        farinosité normale | | |
| date de floraison = W 182 E - 2 jours | | | | |
| Description qualitative | | | | |
| Codes | | | | |
| GEVES | UPOV | Caractères | Notes | Commentaires |
| 866 | 03 | ANGLE FEUILLE | 3 | Petit (5 à 37°) |
| 869 | 04 | ATTITUDE FEUILLE | 3 | Légèrement incurvée |
| 448 | 05 | ZIG-ZAG | 1 | Nul à très faible |
| 899 | 07 | FLORAISON MÂLE | 3 | Précoce |
| 904 | 08 | PIGMENTATION BOURRELET | 3 | Faible |
| 901 | 09 | PIGMENTATION CORPS GLUME | 1 | Nulle à très faible |
| 810 | 10 | PIGMENTATION ANTHERES | 1 | Nulle à très faible |
| 905 | 15 | FLORAISON FEMELLE | 3 | Précoce |
| 446 | 16 | PIGMENTATION SOIES | 1 | Nulle à très faible |
| 447 | 17 | INTENSITE PIGMENTATION SOIES | 1 | Très faible |
| 831 | 11 | COMPACITE BRIN MAITRE | 5 | Moyen |
| 974 | 12 | ANGLE AXE CENTRAL - RAMIFICATION | 5 | Moyen |
| 871 | 13 | ATTITUDE PANICULE | 3 | Légèrement incurvée |
| 937 | 14 | NOMBRE DE RAMIFICATIONS | 3 | Petit |
| 929 | 18 | PIGMENTATION GAINE | 1 | Nulle à très faible |
| 808 | | PIGMENTATION ENTRE-NOEUDS | 1 | Nulle à très faible |
| 933 | 06 | PIGMENTATION RACINES | 1 | Nulle à très faible |
| 814 | 25 | LONGUEUR PEDONCULE | 5 | Court |

(suite)

| Description qualitative | | | | |
|---|---|---|---|---|
| Codes | | | | |
| GEVES | UPOV | Caractères | Notes | Commentaires |
| 920 | | LONGUEUR SPATHES | 5 | Moyen |
| 816 | 28 | FORME EPI | 3 | Cylindrique |
| 818 | 30 | TYPE DE GRAIN | 1 | Corné |
| 922 | 31 | COLORATION SOMMET DU GRAIN | 9 | Noir Bleu |
| 923 | 32 | COLORATION FLAN DU GRAIN | 9 | Noir Bleu |
| 821 | 33 | PIGMENTATION RAFLE | 1 | Absente |
| 874 | 34 | INTENSITE PIGMENTATION RAFLE | 1 | Nulle |
| Description quantitative | | | | |
| 914 | 22-1 | HAUTEUR PLANTE | 5 | Moyenne |
| 918 | 23 | HAUTEUR EPI | 5 | Moyenne |
| 928 | 24 | LARGEUR DU LIMBE | 5 | Moyenne |
| 975 | 19 | LONGUEUR AXE RAMIFICATION INFERIEURE | 5 | Moyen |
| 976 | 20 | LONGUEUR AXE RAMIFICATION SUPERIEURE | 5 | Moyen |
| 921 | 29 | NOMBRE DE RANG | 3 | Petit |
| 838 | 26 | LONGUEUR EPI | 5 | Petit |
| 839 | 27 | DIAMETRE EPI | 3 | Petit |

**Description détaillée de PK6-cherry.**

| Description de PK6 | | | | |
|---|---|---|---|---|
| Albumen | | Allèle y     présence de carotène<br>Allèle fl0$^+$    farinosité normale | | |
| date de floraison = W 182 E - 2 jours | | | | |
| Description qualitative | | | | |
| Codes | | | | |
| GEVES | UPOV | Caractères | Notes | Commentaires |
| 866 | 03 | ANGLE FEUILLE | 3 | Petit (5 à 37°) |
| 869 | 04 | ATTITUDE FEUILLE | 3 | Légèrement incurvée |
| 448 | 05 | ZIG-ZAG | 2 | Faible |
| 899 | 07 | FLORAISON MÂLE | 3 | Précoce |
| 904 | 08 | PIGMENTATION BOURRELET | 7 | Fort |
| 901 | 09 | PIGMENTATION CORPS GLUME | 5 | Moyenne |
| 810 | 10 | PIGMENTATION ANTHERES | 1 | Très faible |
| 905 | 15 | FLORAISON FEMELLE | 3 | Précoce |
| 446 | 16 | PIGMENTATION SOIES | 1 | Nulle à très faible |

(suite)

| Description qualitative | | | | | |
|---|---|---|---|---|---|
| Codes | | | | | |
| GEVES | UPOV | Caractères | | Notes | Commentaires |
| 447 | 17 | INTENSITE PIGMENTATION SOIES | | 1 | Très faible |
| 831 | 11 | COMPACITE BRIN MAITRE | | 5 | Moyen |
| 974 | 12 | ANGLE AXE CENTRAL - RAMIFICATION | | 5 | Moyen |
| 871 | 13 | ATTITUDE PANICULE | | 3 | Légèrement incurvée |
| 937 | 14 | NOMBRE DE RAMIFICATIONS | | 3 | Petit |
| 929 | 18 | PIGMENTATION GAINE | | 3 | Faible |
| 808 | | PIGMENTATION ENTRE-NOEUDS | | 5 | Moyenne |
| 933 | 06 | PIGMENTATION RACINES | | 1 | Nulle à très faible |
| 814 | 25 | LONGUEUR PEDONCULE | | 5 | Court |
| 920 | | LONGUEUR SPATHES | | 5 | Moyen |
| 816 | 28 | FORME EPI | | 2 | Cylindro-conique |
| 818 | 30 | TYPE DE GRAIN | | 1 | Corné |
| 922 | 31 | COLORATION SOMMET DU GRAIN | | 9 | Noir Bleu |
| 923 | 32 | COLORATION FLAN DU GRAIN | | 9 | Noir Bleu |
| 821 | 33 | PIGMENTATION RAFLE | | 9 | Très forte |
| 874 | 34 | INTENSITE PIGMENTATION RAFLE | | 9 | Très forte |
| Description quantitative | | | | | |
| 914 | 22-1 | HAUTEUR PLANTE | | 5 | Moyenne |
| 918 | 23 | HAUTEUR EPI | | 5 | Moyenne |
| 928 | 24 | LARGEUR DU LIMBE | | 5 | Moyenne |
| 975 | 19 | LONGUEUR AXE RAMIFICATION INFERIEURE | | 5 | Moyen |
| 976 | 20 | LONGUEUR AXE RAMIFICATION SUPERIEURE | | 5 | Moyen |
| 921 | 29 | NOMBRE DE RANG | | 3 | Petit |
| 838 | 26 | LONGUEUR EPI | | 5 | Petit |
| 839 | 27 | DIAMETRE EPI | | 3 | Petit |

**EXEMPLE 2: UTILISATION DE LA LIGNEE PK6 COMME LIGNEE MALE INDUCTRICE**

**Pollinisation et obtention des grains haploïdes**

Des plants hybrides F1 d'origines variées, ainsi que des plants de deux lignées synthétiques de maïs sont pollinisés par des inducteurs d'haploïdes PK6. Les plants utilisés présentent, soit un génotype denté (tableau Ia), soit un génotype corné (tableau Ib). La pollinisation est effectuée en parcelle d'isolement ou manuellement. Dans les deux cas, il est recommandé d'effectuer une castration du matériel utilisé comme parent femelle. Cette castration permet une meilleure réussite des fécondations et évite toute pollution par le pollen environnant lors des opérations de pollinisation.

Les grains issus de cette pollinisation sont examinés afin de s'assurer de l'intégrité de la fécondation par PK6. En effet, la lignée PK6 possède un système génétique de coloration de la couche à aleurone du grain (hérité de la lignée WS14). La présence de grains non colorés indique une fécondation indésirable émanant, soit d'un pollen extérieur au dispositif, soit d'un pollen émis par des plantes femelles mal castrées. La présence de grains de coloration très contrastée (jaunes, orangés, blancs ...) distribués de façon anarchique par rapport au bleu-noir de PK6 indique également une pollution par un pollen étranger.

Toutefois, cette vérification ne peut pas être réalisée lorsque le parent femelle possède un inhibiteur de coloration. Cette inhibition se manifeste, soit par l'absence totale de coloration bleue (inhibition totale), soit par un gradient de coloration des grains allant du bleu au jaune (inhibition partielle).

## Obtention des plantules haploïdes

Des semis sont effectués à partir de la totalité des grains dans des plaquettes à 96 alvéoles sur un support de vermiculite, à raison d'un grain par alvéole ; la vermiculite est ensuite recouverte de terreau. La germination s'effectue en serre ou sous abri ; les semis reçoivent des arrosages fréquents.

Le tri des plantules haploïdes s'effectue après le semis, soit sur la base de l'expression d'un caractère récessif présent chez la plante mère (caractère glossy ; tableau Ia), soit sur celle de leur morphologie (tableau Ib).

Les résultats observés sont résumés dans les Tableaux Ia et Ib ci-après :

Le pourcentage d'induction est calculé selon la formule suivante :

$$\text{Pourcentage d'induction} = (\text{nombre d'haploïdes identifiés/nombre de grains germés}) * 100$$

## Doublement chromosomique et culture des plantes traitées

Les plantules haploïdes sélectionnées sont repiquées individuellement dans des plaques alvéolées à 51 godets sur support terreau. Le traitement par la colchicine s'effectue sur les plantules sélectionnées au stade 5 à 6 feuilles ligulées. Le jour précédent le traitement, les plantes ne sont pas arrosées. Après regroupement des plantules, le traitement s'effectue par immersion de leur base jusqu'à la moitié de la gaine de la première feuille, dans une solution aqueuse de colchicine à 1,5g/l. L'immersion est maintenue durant 3 heures et à température ambiante (20°C) (en condition de luminosité normale). Les jeunes plantes sont ensuite repiquées en pots dans un mélange tourbe/terreau en attente de prise de végétation. Les plantes viables sont transférées, l'hiver sous serre en pots de 12 litres sur substrat de pouzzolane + tourbe ou en pleine terre, et l'été sous tunnel plastique ou au champ. Elles sont arrosées avec une solution nutritive.

## Autofécondation

Dès l'apparition des stigmates, on réalise une autofécondation que l'on répète tant qu'il y a émission de pollen. La répétition de cette opération d'autofécondation permet d'augmenter les chances de faire coïncider un anthérozoïde et une oosphère viable. En moyenne 50% à 80% des plantes produisent du pollen. La présence du pollen constitue un indicateur d'un doublement chromosomique réussi au niveau de la panicule (il n'existe pas d'indicateur au niveau des ovules). En l'absence de stress importants, il y a production de grains dans 20 à 60 % des cas alors qu'une plante haploïde émet presque systématiquement des soies. Pour éviter l'attaque des pucerons et la pourriture des grains, il est souhaitable d'ouvrir les spathes trois semaines après la dernière autofécondation. Le nombre moyen de grains par épi est relativement faible (3 à 10) et peut varier de 1 à 50.

Les résultats sont résumés dans les Tableaux Ia et Ib.

La descendance obtenue représente le nombre de plantes produisant un épi.

Le pourcentage de descendance est calculé selon la formule suivante :

$$\text{Pourcentage descendance} = (\text{descendance obtenue/ nombre de plantules haploïdes identifiées}) \times 100.$$

Tableau la

| Génotypes "Dentés" : identification des haploïdes avec le caractère glossy | | | | | |
|---|---|---|---|---|---|
| Matériel génétique pollinisé par la lignée PK 6 | Plantes germées | Plantules haploïdes identifiées | Pourcentage d'induction | Descendance obtenue | Pourcentage descendance |
| ALDGT13 × F1819 | 1152 | 77,00 | **6,7** | 19 | **24,7** |
| F1819 x F816 | 288 | 30,00 | **10,4** | 3 | **10,0** |
| ALDGT13 × F1891 | 1632 | 85,00 | **5,2** | 8 | **9,4** |
| F1819 × F1891 | 1536 | 32,00 | **2,1** | 13 | **40,6** |
| F1891 × F1853 | 480 | 20,00 | **4,2** | 8 | **40,0** |
| F1891 × F1854 | 1478 | 81,00 | **5,5** | 9 | **11,1** |
| SAF6 × F1800 | 384 | 20,00 | **5,2** | 2 | **10,0** |
| SAF6 × F1819 | 672 | 66,00 | **9,8** | 5 | **7,6** |
| SAF8 × F1819 | 3072 | 164,00 | **5,3** | 50 | **30,5** |
| SAF17 × F1819 | 2880 | 200,00 | **6,9** | 12 | **6,0** |
| Pop Dentée 1 | 672 | 38,00 | **5,7** | 3 | **7,9** |
| Pop Dentée 2 | 2304 | 117,00 | **5,1** | 9 | **7,7** |
| LPO1 × F1819 | 2880 | 64,00 | **2,2** | 15 | **23,4** |
| LPO1 × F1891 | 768 | 42,00 | **5,5** | 2 | **4,8** |
| **Moyenne** | **1442,7** | **74,0** | **5,7** | **11,3** | **16,7** |

Tableau Ib

| Génotypes "Cornés": identification des haploïdes selon la morphologie (plus aléatoire) | | | | | |
|---|---|---|---|---|---|
| Matériel génétique pollinisé par la lignée PK 6 | Plantes germées | Plantules haploïdes identifiées | Pourcentage d'induction | Descendance obtenue | Pourcentage descendance |
| D171 * F810 | 1883 | 105 | **5,6** | 17 | **16,2** |
| F878 * D171 | 1360 | 86 | **6,3** | 19 | **22,1** |
| D171 * F336 | 1860 | 113 | **6,1** | 24 | **21,2** |
| F4 * 258 | 920 | 28 | **3,0** | 2 | **7,1** |
| EP1.F252.M1 * D171 (F2) | 1702 | 55 | **3,2** | 6 | **10,9** |
| D171 * EP1.F252.M1 (F2) | 1790 | 98 | **5,5** | 12 | **12,2** |
| D171 * EP1.F252.M1 (F1) | 1718 | 56 | **3,3** | 4 | **7,1** |
| SynthétiqueFP141F1 | 1741 | 61 | **3,5** | 10 | **16,4** |
| SynthétiqueFP141F2 | 1650 | 82 | **5,0** | 6 | **7,3** |
| **Moyenne** | **1624,9** | **76** | **4,6** | **11,1** | **13,4** |

Malgré un pourcentage identique de plantes produisant du pollen, le taux de descendance obtenu à partir de matériel corné européen est en moyenne inférieur à celui obtenu à partir du matériel denté américain.

8

## EXEMPLE 3: UTILISATION DE LA LIGNEE PK6-CHERRY COMME LIGNEE MALE INDUCTRICE

Des plants issus d'une population de maïs dentée d'origine nord américaine à base très large (pop1), et de sous-populations de pop1 obtenues après différents cycles de sélection (pop2 à pop5), sont pollinisés par des inducteurs d'haploïdes PK6-cherry.

La pollinisation et la récolte des grains, ainsi qu'un premier tri des haploïdes potentiels sur la base de la coloration du grain sont effectués comme indiqué dans l'exemple 2. Des semis sont réalisés à partir de la totalité des grains comme indiqué dans l'exemple 2.

Le tri des plantules s'effectue dès le stade 3 à 4 feuilles visibles. Les plantules diploïdes présentent une nette coloration rouge du joint foliaire au niveau de la première feuille. Cette coloration du joint foliaire est également visible parfois au niveau de la seconde feuille. En revanche, les plantules haploïdes possèdent un joint foliaire vert.

Les résultats sont résumés dans le Tableau II.

Le pourcentage d'induction est déterminé comme suit :

Pourcentage d'induction = (nombre d'haploïdes

identifiés/nombre de grains germés) * 100.

Tableau II

| Matériel génétique pollinisé par PK 6-cherry | Pourcentage d'induction |
|---|---|
| Pop 1 (CO) | 5,6 |
| Pop 2 (COHD) | 11,6 |
| Pop 3 (C1S1) | 7,1 |
| Pop 4 (C2S1) | 8,6 |
| Pop 5 (C1HD) | 12,8 |
| Total | 9,12 |

## EXEMPLE 4: COMPARAISON DES PROPRIETES INDUCTRICES DE LA LIGNEE PK6 ET D'AUTRES LIGNEES MALES INDUCTRICES DE L'ART ANTERIEUR

Des plants femelles de différentes lignées de maïs sont pollinisés par des mâles inducteurs d'haploïdes gynogénétiques des lignées Stock6, WS14, FIGH1 ou PK6. La pollinisation, la récolte des grains, et la sélection des plants haploïdes s'effectuent comme décrit dans l'exemple 2.

Le pourcentage d'induction est calculé selon la formule suivante :

Pourcentage d'induction = (nombre d'haploïdes

identifiés/nombre de grains semés) * 100

Les résultats sont illustrés dans le Tableau III.

Tableau III

| Matériel génétique pollinisé (lignées) | Pourcentage d'induction d'haploïdes gynogénétiques | | | |
|---|---|---|---|---|
| | Stock 6 | WS14 | FIGH1 | PK 6 |
| W64A | 2,2 | 2,8 | 1,4 | 4,1 |
| F16 | 2 | 3,1 | 1,2 | 4,2 |
| Co220 | 1,8 | 3,4 | 1 | 5,4 |
| F1254 | 1,6 | 2,7 | 1,3 | 4,8 |

Tableau III   (suite)

| Matériel génétique pollinisé (lignées) | Pourcentage d'induction d'haploïdes gynogénétiques | | | |
|---|---|---|---|---|
| | Stock 6 | WS14 | FIGH1 | PK 6 |
| F2 | 1,5 | 3,2 | 1,8 | 5 |
| B73 | 1,3 | 1,8 | 1 | 3,5 |
| Mo17 | 1,3 | 1,8 | 1,3 | 3,1 |
| W23 | 1,3 | 2,4 | 1,8 | 6,2 |
| A632 | 1,3 | 1,9 | 0,5 | 3,7 |
| A619 | 1,1 | 0,9 | 1,5 | 5 |
| Cl35 | 0,9 | 1,7 | 0,6 | 6 |
| Co255 | 0,8 | 2,5 | 3 | 5,2 |
| F492 | 0,7 | 1,5 | 2 | 4,1 |
| F1444 | 0,7 | 2,8 | 2,4 | 5,4 |
| MBS847 | 0,4 | 1,3 | 0,7 | 4 |
| F186 | 0,3 | 1,2 | 1,1 | 3,4 |
| Moyenne lignées | 1,20% | 2,20% | 1,40% | 4,60% |

Ces résultats montrent que la fréquence moyenne d'induction d'haploïdes gynogénétiques obtenue avec la lignée PK 6 est plus de 2 fois supérieure à celle obtenue avec la lignée inductrice la plus efficace décrite dans l'art antérieur, à savoir WS14.

**Revendications**

1.  Procédé d'obtention d'haploïdes de maïs, comprenant une étape de pollinisation du matériel femelle par une plante d'une lignée inductrice d'haploïdes gynogénétiques et une étape de sélection des haploïdes induits par cette pollinisation, **caractérisé en ce que** la lignée inductrice d'haploïdes gynogénétiques correspond à la lignée PK6, déposée le 19/02/2003 auprès de la NCIMB sous le numéro d'accès NCIMB 41160, ou à une lignée dérivée.

2.  Procédé selon la revendication 1, **caractérisé en ce que** ladite lignée dérivée est la lignée PK6-cherry déposée le 19/02/2003 auprès de la NCIMB sous le numéro d'accès NCIMB 41161.

3.  Procédé d'obtention de double-haploïdes de maïs, **caractérisé en ce qu'**il comprend une étape de doublement chromosomique de plantes haploïdes obtenues par le procédé selon une quelconque des revendications 1 ou 2.

4.  Procédé selon la revendication 3 **caractérisé en ce que** l'étape de doublement chromosomique comprend un traitement à la colchicine.

5.  Procédé selon une quelconque des revendications 3 ou 4, **caractérisé en ce qu'**il comprend une étape d'autofécondation des plantes ayant subi l'étape de doublement chromosomique.

6.  Utilisation de la lignée PK6 ou d'une lignée dérivée en tant que lignée inductrice d'haploïdes gynogénétiques chez le maïs.

7.  Utilisation selon la revendication 6, **caractérisée en ce que** ladite lignée dérivée est la lignée PK6-cherry.

**EP 1 495 671 A1**

<table>
<tr><td colspan="2">Office européen<br>des brevets</td><td>**RAPPORT DE RECHERCHE EUROPEENNE**</td><td>Numéro de la demande<br>EP 03 29 1693</td></tr>
</table>

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EDER J ET AL: "In vivo haploid induction in maize"<br>THEORETICAL AND APPLIED GENETICS,<br>vol. 104, no. 4, mars 2002 (2002-03),<br>pages 703-708, XP002265935<br>ISSN: 0040-5752<br>* le document en entier *<br>--- | 1-7 | A01H1/08 |
| X,D | BORDES J ET AL: "Haplodiploidization of maize (Zea mays L.) through induced gynogenesis assisted by glossy markers and its use in breeding"<br>AGRONOMIE (PARIS),<br>vol. 17, no. 5, juin 1997 (1997-06), pages 291-297, XP008025907<br>ISSN: 0249-5627<br>* le document en entier *<br>--- | 1-7 | |
| X,D | LASHERMES P ET AL: "GENETIC CONTROL OF MATERNAL HAPLOIDY IN MAIZE ZEA-MAYS L. AND SELECTION OF HAPLOID INDUCING LINES"<br>THEORETICAL AND APPLIED GENETICS,<br>vol. 76, no. 3, 1988, pages 405-410,<br>XP008025902<br>ISSN: 0040-5752<br>* le document en entier *<br>----- | 1-7 | **DOMAINES TECHNIQUES RECHERCHES (Int.Cl.7)**<br><br>A01H |

Le présent rapport a été établi pour toutes les revendications

| Lieu de la recherche | Date d'achèvement de la recherche | Examinateur |
|---|---|---|
| LA HAYE | 2 janvier 2004 | Oderwald, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)